# EUROPEAN PATENT SPECIFICATION

(11) **EP 4 263 861 B1**
(45) Date of publication and mention of the grant of the patent: **08.04.2026**
(21) Application number: 21854940.0
(22) Date of filing: 21.12.2021
(51) Int. Cl.: C12Q 1/6844

(54) **COLORIMERIC METHOD FOR DETECTION OF AMPLIFIED GENETIC MATERIAL AND USES THEREOF**
KOLORIMERES VERFAHREN ZUM NACHWEIS VON AMPLIFIZIERTEM GENETISCHEM MATERIAL UND VERWENDUNGEN DAVON
MÉTHODE COLORIMÉTRIQUE POUR LA DÉTECTION D'UN MATÉRIEL GÉNÉTIQUE AMPLIFIÉ ET UTILISATIONS ASSOCIÉES

(30) Priority: 21.12.2020 PT 2020116962
(43) Date of publication of application: 25.10.2023
(73) Proprietor: UNIVERSIDADE NOVA DE LISBOA, 1099-085 Lisboa (PT)
(72) Inventor: RIBEIRO PIMENTEL, Catarina Isabel, 2780-051 Oeiras (PT); SÁ DE ALMEIDA AMARAL, Catarina, 3520-167 Viseu (PT); BARROSO PEREIRA LIMA, Luís Miguel, 1500-021 Lisboa (PT)
(74) Representative: Patentree
(86) International application number: PCT/IB2021/062122
(87) International publication number: WO 2022/137130

(56) References cited:
- WO-A1-2019/234252
- SHAMSIPUR MOJTABA: "SPECTROPHOTOMETRIC STUDY OF COBALT, NICKEL, COPPER, ZINC, CADMIUM AND LEAD COMPLEXES WITH MUREXIDE IN DIMETHYLSULPHOXIDE SOLUTION", TALANTA, vol. 39, no. 9, 1 January 1992 (1992-01-01), pages 1209 - 1212, XP055915240
- AMARAL CATARINA ET AL: "A molecular test based on RT-LAMP for rapid, sensitive and inexpensive colorimetric detection of SARS-CoV-2 in clinical samples", SCIENTIFIC REPORTS, vol. 11, no. 1, 12 August 2021 (2021-08-12), XP055915179, Retrieved from the Internet <URL:https://www.nature.com/articles/s41598-021-95799-6> DOI: 10.1038/s41598-021-95799-6

## Description

### TECHNICAL FIELD

The present disclosure relates to the field of laboratorial analysis, in particular for medical diagnostic, in particular to a colorimetric method based on a complexometric indicator, for detecting genetic material after an amplification reaction as defined in claim 1. In particular, the disclosure relates to a colorimetric method for detecting genetic material after a loop-mediated isothermal amplification reaction.

### BACKGROUND

A robust population-scale testing strategy for SARS-CoV-2 based on rapid, reliable, decentralized and affordable diagnostic tests is of utmost priority to guide public health interventions.

The gold standard of COVID-19 testing is RT-PCR (RT-PCR), which detects the genetic material of SARS-CoV-2 in nasopharyngeal (NP) samples. Although very reliable, RT-PCR diagnostics are complex, laborious and expensive, and its worldwide use caused, in the beginning of the pandemic, a shortage of reagents needed for sample collection and viral RNA extraction. Thus, RT-PCR-based tests, in their current format, are unlikely to serve the purposes of a mass screening of the virus.

Loop-mediated isothermal amplification (LAMP) is a DNA amplification method that allows rapid and sensitive detection of a specific gene [1-3]. LAMP merged with reverse transcription (RT-LAMP) has been successfully used for the detection of several respiratory RNA viruses [4-8], including SARS-CoV-2 (reviewed in [9]). RT-LAMP is a powerful alternative to RT-PCR due to its high specificity and sensitivity, cost-effectiveness, and fast turnaround time (typically 30 minutes). In RT-LAMP, the amplification of the genetic material of the virus occurs at a constant temperature and, therefore, diagnostic tests based on RT-LAMP can be carried out anywhere with basic resources, as they only require a heat block or a water bath set to a single temperature. The reaction products can be analyzed by means of conventional DNA-intercalating dyes, agarose gel electrophoresis, UV-light illumination, or real-time fluorescence [10]. Alternatively, end-point colorimetric readouts are also possible through the detection of reaction by-products, such as pyrophosphate and protons, which are released during DNA polymerization, after the incorporation of deoxynucleotide triphosphates. LAMP colorimetric methods detect the turbidity, triggered by the accumulation of magnesium pyrophosphate [1], or color changes, occurring when complexometric indicators [3,11], pH sensitive dyes [12] or even DNA-intercalating dyes [13-15] are incorporated into the reaction. The simple technical and instrumental requirements of colorimetric RT-LAMP tests make them extremely attractive for point-of-care (POC) use and implementation in low-resource settings.

Colorimetric RT-LAMP has been successfully used for detection of SARS-CoV-2 in NP fluids from COVID-19 patients [15-24].

Recently, it was shown that SARS-CoV-2 could be detected in the saliva of infected individuals, highlighting salivary tests as valuable alternatives for COVID-19 diagnosis [25-27]. Saliva-based testing has numerous advantages over NP sampling, especially in a mass screening scenario. It can be performed easily and non-invasively, thus minimizing patient discomfort, and it does not require specialized personnel or the use of protective equipment, which saves time and reduces costs. For these reasons, saliva RT-PCR and RT-LAMP tests for SARS-CoV-2 detection have been widely explored in recent months [28-31]. Document WO2019234252A1 relates to methods for detecting a tandem repeat in a nucleic acid sequence under isothermal conditions using primers.

Document Shamsipur Moitaba: "SPECTROPHOTOMETRIC STUDY OF COBALT, NICKEL, COPPER, ZINC, CADMIUM AND LEAD COMPLEXES WITH MUREXIDE IN DIMETHYLSULPHOXIDE SOLUTION", Talanta, vol. 39, no. 9, 1 January 1992 (1992-01-01), pages 1209-1212, XPO55915240, relates to the spectrophotometric study of metal-ligand complexation, specifically describing the formation of 1:1 complexes between murexide and divalent metal ions such as cobalt, nickel, copper, zinc, cadmium, and lead in dimethylsulphoxide solution.

These facts are disclosed in order to illustrate the technical problem addressed by the present disclosure.

### GENERAL DESCRIPTION

The present disclosure relates to a colorimetric method for a detection of an amplified genetic material in a biological sample, comprising the following steps:; amplifying the genetic material of the biological sample to obtain a solution of amplified genetic material; adding a detection ensemble comprising murexide and zinc(II) chloride to the solution, wherein the concentration of the zinc(II) chloride ranges from 0.4 to 3 mM; and evaluating the color change of the solution. In another embodiment, other zinc salts or ligands can be used to form a complex with murexide, i.e., the complex forms a detection ensemble.

In an embodiment, the amplification of the genetic material comprises a polymerization step with production of pyrophosphate.

In an embodiment, the detection ensemble may comprise murexide and a zinc(II) inorganic salt. In another embodiment, the zinc(II) compound is selected from a list comprising: zinc(II) chloride (ZnCl₂), zinc(II) sulphate (ZnSO₄), zinc(II) nitrate Zn(NO₃)₂, zinc(II) perchlorate (Zn(ClO₄)₂), zinc(II) acetate (Zn(CH₃COO)₂), or any other inorganic zinc(II) salt. Preferably, the zinc(II) compound is zinc(II) chloride (ZnCl₂).

In an embodiment, the detection ensemble may comprise murexide and zinc(II) chloride (ZnCl₂).

In an embodiment, the zinc(II) compound may be a complex with an organic ligand selected from a list comprising: dipicolinic acid, dipicolylamine, dipicolylamine derivative, any other polyamine compound, or mixtures thereof.

In another embodiment, the detection ensemble may comprise murexide and a zinc(II) complex of an organic ligand such as dipicolinic acid.

In another embodiment, the detection ensemble may comprise murexide and a zinc(II) complex of an organic polyamine such as dipicolylamine (DPA).

In another embodiment, the detection ensemble may comprise murexide and a zinc(II) complex of a DPA derivative containing any additional chemical function appended on the aliphatic nitrogen atom of DPA.

In another embodiment, the detection ensemble may comprise murexide and a zinc(II) complex of a DPA derivative containing any additional chemical functions appended on the pyridine groups of DPA.

In yet another embodiment, the detection ensemble may comprise murexide and a zinc(II) complex of a DPA derivative containing both an additional chemical function appended on the aliphatic nitrogen atom of DPA and additional chemical functions appended on the pyridine groups of DPA.

In an embodiment, the additional chemical function may be selected from a list comprising: bromophenyl, benzyl, alkyl, alkenyl, alkynyl, phenyl, hydroxyl, carbonyl, aldehyde, or carboxylate.

In another embodiment, the genetic material may be selected from viral, bacterial, fungal, protozoal parasite, plant genetic material, or human genetic material. In a further embodiment, the genetic material is viral genetic material.

In an embodiment, the viral genetic material may be from a coronavirus, influenza virus, or zika virus. In another embodiment, the viral genetic material is from SARS-CoV-2.

In an embodiment, the amplification step may comprise the addition of a polymerase. In a further embodiment, the polymerase may be selected from a list consisting of: Bst1, Bst2, Bst3, Taq, Pfu, Pfx, or mixtures thereof.

In another embodiment, the biological sample may be a saliva sample, nasopharyngeal sample, blood sample, or plant tissues sample. Preferably, the biological sample is a saliva sample.

In an embodiment, the amplifying step may be an isothermal amplification. In an embodiment for better results, the amplifying step may be performed by loop-mediated isothermal amplification reaction. In a further embodiment, the amplifying step is performed by reverse transcription loop-mediated isothermal amplification reaction.

In another embodiment, the amplifying step may be performed by polymerase chain reaction (PCR).

In an embodiment for better results, the murexide concentration may range from 0.3 to 0.7 mM. In yet another embodiment, the murexide concentration may range from 0.4-0.5 mM.

In an embodiment for better results, the concentration of the zinc(II) compound may range from 1 to 3 mM; preferably ZnCl₂. In another embodiment, the concentration of the zinc(II) compound concentration may range from 1.5 to 2.5 mM, preferably ZnCl₂.

In an embodiment for better results, the method may further comprise an RNA or DNA extraction step before the amplification step.

In an embodiment, the colorimetric method may further comprise a step of incubating the solution of amplified genetic material.

In an embodiment, the colorimetric method of the present disclosure may comprise obtaining a biological sample; amplifying a viral genetic material by reverse transcription loop-mediated isothermal amplification reaction; incubating the reaction sample, preferably at 60 - 70 °C for 10 to 90 minutes; adding murexide and ZnCl₂; evaluating the color change of the sample.

In an embodiment for better results, the incubation temperature may range from 60-100 °C, preferably from 60-80 °C.

In an embodiment for better results, the incubation time may range from 10 to 90 minutes, preferably from 20-60 minutes, more preferably 30 minutes.

In an embodiment for better results, the limit of detection may be from 10 to 100 viral copies per reaction, preferably 25 viral copies per reaction.

Another aspect of the present disclosure relates to the use of a kit for detection of an amplified genetic material in a biological sample by the described method, wherein the kit comprises murexide and a zinc(II) compound , wherein the concentration of the zinc(II) chloride ranges from 0.4 to 3 mM, preferably wherein the zinc(II) compound is ZnCl₂.

The present disclosure relates to the use of the detection ensemble comprising murexide and a zinc(II) compound as an indicator for detection of an amplified genetic material in a biological sample, wherein the concentration of the zinc(II) chloride ranges from 0.4 to 3 mM .

In an embodiment, the amplified genetic material is detected by the release of pyrophosphate in the samples in which amplification occurred. The presence of pyrophosphate from amplification of genetic material is indicated by the color change of the indicator murexide, which presents different colors when it is bound to zinc(II) and when not.

In an embodiment, the disclosed detection ensemble comprises murexide bound to a zinc(II) compound. Pyrophosphate is able to displace murexide from binding to the zinc(II) compound because the interaction between pyrophosphate and zinc(II) is stronger than the interaction between murexide and zinc(II).

Another aspect of the present disclosure relates to the use of a microfluidic diagnostic cartridge or a lab-on-chip device for detection of an amplified genetic material in a biological sample by the disclosed colorimetric method, , wherein the microfluidic diagnostic cartridge or a lab-on-chip device comprises murexide and zinc(II) chloride as an indicator for detection of an amplified genetic material in a biological sample, wherein the concentration of the zinc(II) chloride ranges from 0.4 to 3 mM.

In another embodiment, the disclosed method can be used in combination with any amplification reaction that releases pyrophosphate during DNA polymerization.

In a further embodiment, the method is suitable for the detection of viral genetic material, bacterial genetic material, fungal genetic material, protozoal parasite genetic material or human genetic material.

### BRIEF DESCRIPTION OF THE DRAWINGS

The following figures provide preferred embodiments for illustrating the disclosure and should not be seen as limiting the scope of invention. The examples outside the scope of the claims are comparative examples.

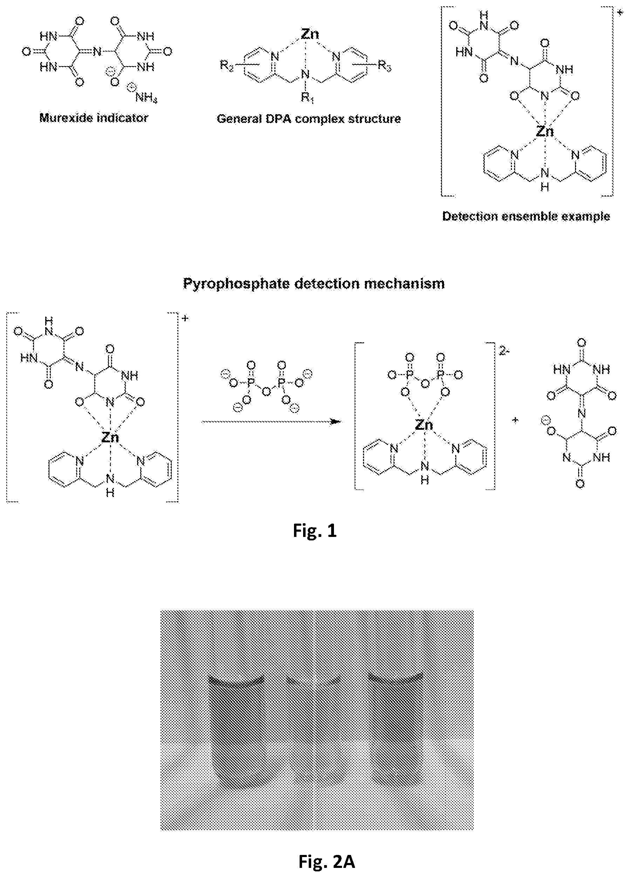

**Figure 1****:** Schematic representation of the colorimetric detection method comprising murexide and a zinc compound.

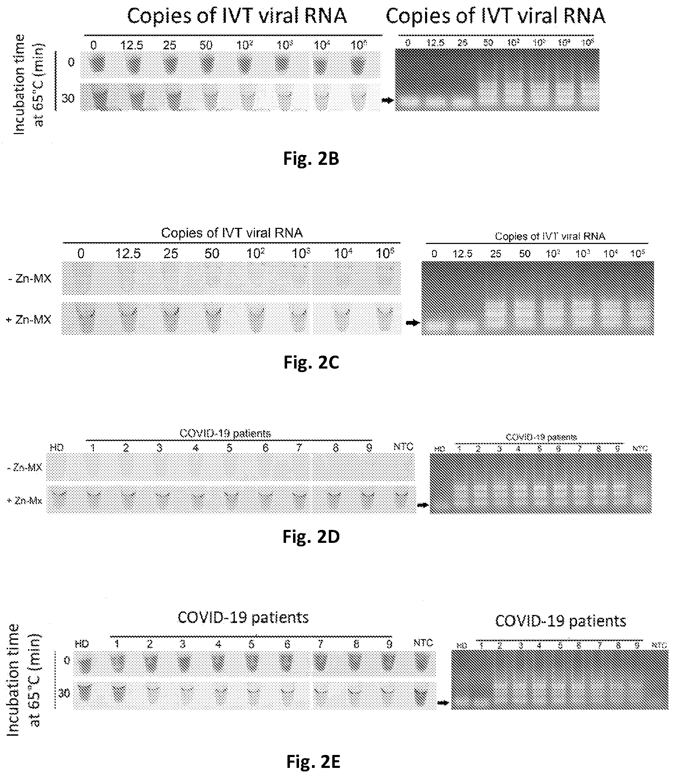

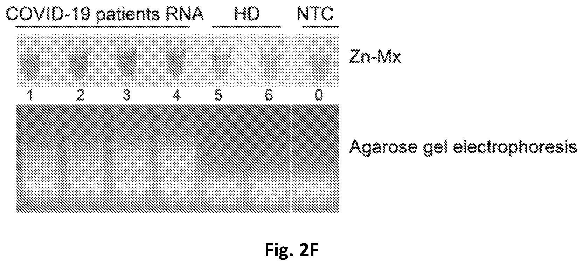

**Figure 2****:** Embodiment of the colorimetric detection method based on the complex murexide-zinc (Zn-MX), as described in Example 1 and Example 7. (A) From Example 1, samples mimicking the components of the LAMP reaction, after adding successively murexide(left), then zinc(II) chloride (center), then pyrophosphate (right). From Example 7, tenfold dilutions of in vitro transcribed (IVT) viral IVT RNA (N-gene) were amplified via RT-LAMP and detected using phenol red (B) or Zn-MX (C). Amplification was confirmed by agarose gel electrophoresis (AGE). Saliva samples of a healthy donor (HD) and of nine COVID-19 patients were analyzed by RT-LAMP followed by detection with Zn-MX (D) or phenol red I and amplification was confirmed by AGE. (F) Viral RNA extracted from NP fluid of a COVID-19 patient was amplified by RT-LAMP and detected using the complex murexide- zinc(II) chloride. HD- healthy donor, NTC-No template control.

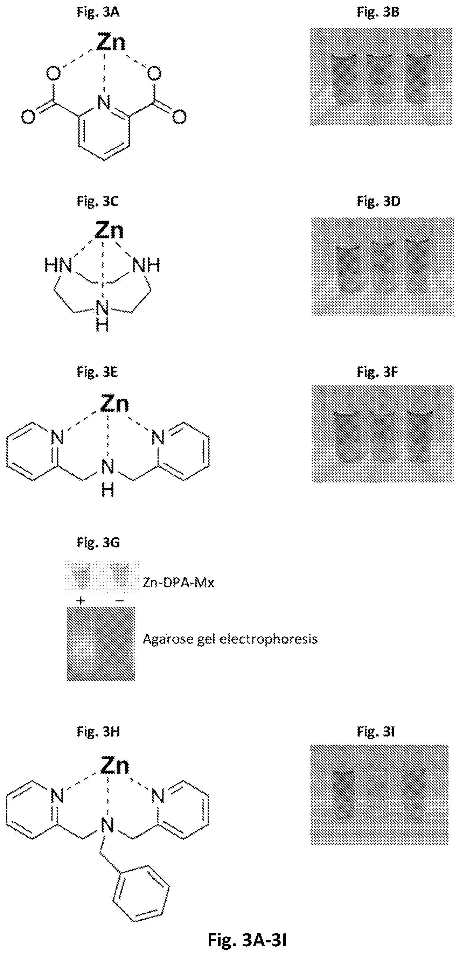

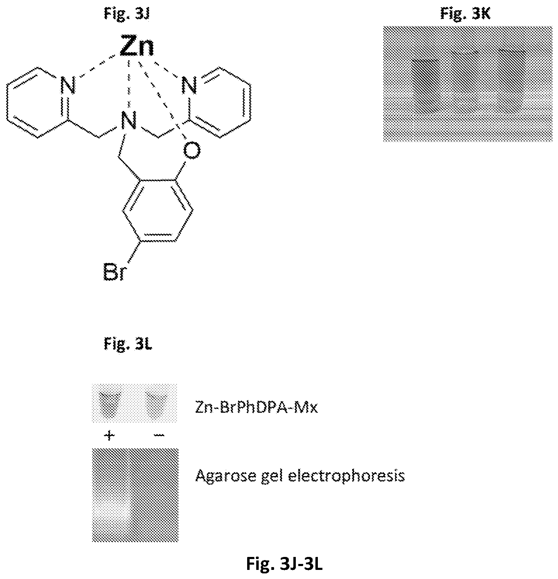

**Figure 3** - Use of other zinc(II) compounds in the colorimetric detection method (Examples 2 and 3). (A) structure of the zinc(II) complex of dipicolinic acid (Zn-DP), and (B) samples mimicking the components of the LAMP reaction after adding successively murexide (left), then the Zn-DP complex (center), then pyrophosphate (right). From Example 3, (C) structure of the zinc(II) complex of 1,4,7-triazacylcononane (Zn-TACN), and (D) samples mimicking the components of the LAMP reaction after adding successively murexide (left), then the Zn-TACN complex (center), then pyrophosphate (right). From Example 4, (E) structure of the zinc(II) complex of dipicolylamine (Zn-DPA), and (F) samples mimicking the components of the LAMP reaction after adding successively murexide (left), then the Zn-DPA complex (center), then pyrophosphate (right). From Example 8, (G) the Zn-DPA complex and murexide (Zn-DPA-Mx) were used for detection of genetic material of the fungus *Candida albicans* amplified by LAMP. "+" indicates addition of C. *albicans* DNA; "-" indicates a negative control (without DNA). Amplification was confirmed by agarose gel electrophoresis. From Example 5, (H) structure of the zinc(II) complex of N-benzyl-dipicolylamine (Zn-BnDPA), and (I) samples mimicking the components of the LAMP reaction after adding successively murexide (left), then the Zn-BnDPA complex (center), then pyrophosphate (right). From Example 6, (J) structure of the zinc(II) complex of N-bromophenol-dipicolylamine (Zn-BrPhDPA), and (K) samples mimicking the components of the LAMP reaction after adding successively murexide (left), then the Zn-BrPhDPA complex (center), then pyrophosphate (right). From Example 11, (L) the Zn-BrPhDPA complex and murexide (Zn-BrPhDPA-Mx) were used for detection of genetic material of the fungus *Candida albicans* amplified by LAMP. "+" indicates addition of *C*. *albicans* DNA; "-" indicates a negative control (without DNA).

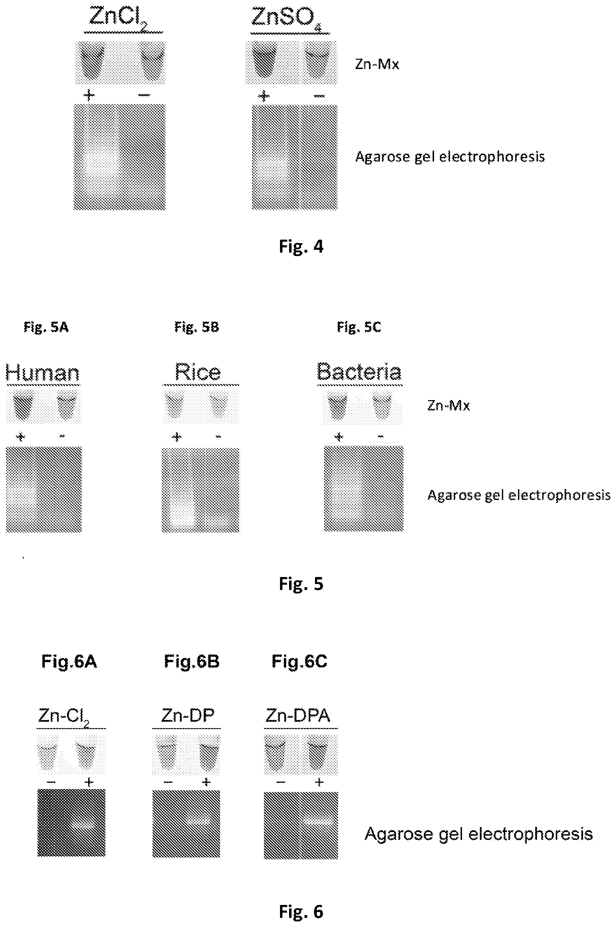

**Figure 4****-** Use of other zinc salts in the colorimetric detection method based on the complex murexide-zinc. From Example 9: ZnCl₂ and ZnSO₄ complexes with murexide (Zn-MX) were used for detection of the genetic material of the fungus *Candida albicans* amplified by LAMP. "+" indicates addition of *C*. *albicans* DNA; "-" indicates a negative control (without DNA). Amplification was confirmed by agarose gel electrophoresis.
**Figure 5** - Detection of the genetic material from different sources using the colorimetric detection method based on the complex murexide-zinc (Zn-Mx), after amplification by LAMP. From Example 10: ZnCl₂ complex with murexide was used for detection of human (A), plant (B) or bacterial (C) genetic material after amplification by LAMP. "+" indicates addition of DNA; "-" indicates a negative control (without DNA). Amplification was confirmed by agarose gel electrophoresis.
**Figure 6** - Detection of genetic material using a different polymerase and the colorimetric detection method based on the complex murexide-zinc. From Example 12: (A) ZnCl₂ complex with murexide was used for detection of genetic material after amplification by PCR. From Example 13: (B) Zn-DP complex with murexide was used for detection of genetic material after amplification by PCR. From Example 14: (C) Zn-DPA complex with murexide was used for detection of genetic material after amplification by PCR."+" indicates addition of DNA; "-" indicates a negative control (without DNA). Amplification was confirmed by agarose gel electrophoresis.

### DETAILED DESCRIPTION

The present disclosure relates to a colorimetric method for a detection of an amplified genetic material in a biological sample, comprising the following steps: obtaining a biological sample; amplifying the genetic material to obtain a solution of amplified genetic material; adding a detection ensemble comprising murexide and a zinc(II) compound to the solution of amplified genetic material; and evaluating the color change of the solution. An aspect of the present disclosure also relates to the use of murexide indicator (5,5-Nitrilodibarbituric acid monoammonium salt, or ammonium purpurate, CAS 3051-09-0) and a zinc(II) compound for detection of an amplified genetic material in a biological sample. A kit, a microfluidic diagnostic cartridge or a lab-on-chip device for detection of an amplified genetic material in a biological sample by the described method is also disclosed.

In an embodiment, the colorimetric method further comprises a step of incubating the solution of amplified genetic material.

In particular, the present disclosure relates to a RT-LAMP colorimetric test for the detection of viral RNA samples, extracted from the NP fluid, or directly from the saliva, of infected patients.

In an embodiment, the test relies on a colorimetric detection method based on a complexometric indicator murexide which is capable of detecting genetic material with great analytical sensitivity after an amplification reaction.

In an embodiment, the amplification reaction is RT-LAMP. The main components of the RT-LAMP colorimetric reaction are two enzymes (a reverse transcriptase (RT) and a strand displacement polymerase), a colorimetric dye (phenol red) and a primer set (typically composed of six primers) [12]. In an embodiment, the genetic material of SARS-CoV-2 was detected using RT-LAMP, with a primer set previously validated *in vitro* by Zhang et al [24] and tested, on clinical specimens from large cohorts of COVID-19 patients [16-19]. The primer set (N-A) targeted the N gene, which encodes the nucleocapsid protein and has the most abundant expression of subgenomic mRNA during infection [32].

In an embodiment, two different assay formats were tested. In one format, it was used the WarmStart Colorimetric LAMP 2× Master Mix (New England Biolabs), which includes all the reagent components with the exception of the primers. In the other, the separate enzymes (RTx and Bst 2.0) were purchased from New England Biolabs, while the reaction buffer and colorimetric dye (phenol red) were prepared in-house as described by Tanner et al. [12]. The analytical sensitivities of these two setups were evaluated and compared by assaying in parallel tenfold serial dilutions of an in vitro transcribed N-gene RNA standard (IVT RNA), starting from 10⁵ copies down to 1 copy (per 20 µL reaction), at tenfold intervals. Color changes from pink (negative) to yellow (positive) were registered after a 30-minute incubation period at 65°C and the amplification products were resolved by agarose gel electrophoresis. Ten replicates were analyzed per assay format and IVT RNA dilutions were simultaneously analyzed by qRT-PCR. The limit of detection (LoD) was reliably found to be between 100-1000 viral copies for the assay using the WarmStart Colorimetric LAMP 2× Master Mix, whereas for the one using the separate components the LoD was consistently one Log10 lower (10-100 copies). However, for half of the replicates, a tenfold lower LoD was achieved for both test formats. Therefore, 100-1000 (WarmStart Colorimetric LAMP 2× Master Mix) and 10-100 (reaction with separate components) was defined as the robust limits of detection.

As comparative example, for the same serial dilution range, the qRT-PCR assay was able to consistently detect down to 10 copies per reaction (mean Ct=37.43). Compared to qRT-PCR, the RT-LAMP assay, depending on the test setup, detected up to ten- or one hundred-fold less copies of viral RNA. As the RT-LAMP format using the separate components was consistently more sensitive, this setup was used in subsequent assays.

In an embodiment, a standard clinical qRT-PCR was used to test a set of surplus RNA samples extracted from the nasopharyngeal (NP) fluid of 177 individuals who were tested for COVID-19, as comparative data. The samples comprised 126 RNA samples that tested positive (qRT-PCR positive, Ct ≤40) and 51 samples that tested negative (qRT-PCR negative, Ct ≥ 40). After incubation for 30 minutes at 65°C, a pink to yellow color change was visualized in all RT-LAMP reactions estimated to have less than 1000 RNA molecules present in the reaction (qRT-PCR positive, Ct ≤31), which is in agreement with the observed experimental sensitivity. Two false positives, i.e., two qRT-PCR negative samples that scored positive in the RT-LAMP assay, were found (Table 1). Thus, the overall specificity of the assay was 96.1% (Cl: 87-99%) and the sensitivity for samples with Ct ≤31 was 100% (Cl: 94.7-100%). For lower viral load, as measured by qRT-PCR (higher Ct values), the assay showed a decrease in diagnostic sensitivity (Table 1).

Overall, these results indicate a robust performance of the colorimetric RT-LAMP assay across a broad range of purified RNA samples.

**Table 1. Embodiment of RT-LAMP sensitivity across different ranges of Ct values.**

| **qRT-PCR** (Ct values) | **RT-LAMP** | | |
|---|---|---|---|
| | **True positives** | **False negatives** | **% Sensitivity (95 % CI)*** |
| **0 - 40** | 84 | 42 | 67.2 (58.56 - 74.81) |
| **0 - 31** | 68 | 0 | 100 (94.65 - 100) |
| **31 - 35** | 17 | 20 | 45.95 (31.04 - 61.62) |
| **35 - 40** | 11 | 27 | 28.95 (17 - 44.76) |

In an embodiment, the RT-LAMP assay was optimized for direct detection of SARS-CoV-2 in saliva samples. To reduce the risk associated with handling samples containing infectious viral particles, saliva was self-collected into a tube and placed at 95°C for 30 minutes, for inactivation. After a brief centrifugation step that significantly improved assay reliability, the supernatant was diluted with TE, to buffer basal pH differences in saliva, and immediately analyzed (2 µl) or stored at -80°C.

In an embodiment, the LoD of the assay using both the IVT RNA standard and viral SARS-CoV-2 particles spiked into healthy human saliva to simulate clinical samples. It was possible to detect 100 IVT RNA copies and 24 SARS-CoV-2 viral particles per reaction in only 30 minutes after inactivation, using the described RT-LAMP protocol.

In an embodiment, saliva and matched NP swab specimens of 49 individuals infected with SARS-CoV-2 (as previously determined by qRT-PCR) were collected and analyzed by RT-LAMP (saliva) and qRT-PCR (NP fluid). In addition, 15 saliva samples of healthy donors were tested by RT-LAMP. Saliva samples were self-collected as described above, and individuals were asked not to eat or drink before testing. A set of 10 COVID-19-positive patients were asked to induce salivation by placing the tongue on the salivary sublingual glands. For this group, SARS-CoV-2 sequences were only detected in the saliva of one patient using the direct RT-LAMP assay. However, after RNA extraction, 8 out of 10 individuals were identified as being SARS-CoV-2-positive. In another embodiment, for all other positive samples where salivation was not induced, a good correlation with the qRT-PCR results was obtained, as 33 out of 39 samples were identified as positive samples, with no false positives registered. Therefore, the direct RT-LAMP assay had a sensitivity of 85% (Cl: 70-93%) and a specificity of 100%, for saliva samples with matched NP swabs with Ct ≤28. Reaction volumes, but not saliva amounts, were scaled up to increase the assay sensitivity. Also, all saliva samples that were falsely negative by direct RT-LAMP were positive after RNA extraction. This step increases by 4-9 times the estimated cost of the assay.

In an embodiment, miniprep columns, which are routinely used in molecular biology laboratories to purify bacterial plasmids, were tested regarding the possibility to be used to extract viral RNA from saliva samples. Surprisingly, all false negative samples were found to be positive after RNA purification using this method.

The strong and evident color shift observed with phenol red renders this pH-sensitive dye much preferred for end-point colorimetric detection of LAMP products. However, when the phenol red method is used with crude samples, interference of the sample pH with the assay readout is often observed. Although several colorimetric indicators are available for detection of LAMP products [3,11-15], the pale color shift they produce, which is difficult to distinguish by the naked eye, has certainly restrained their wide use.

In an embodiment, a colorimetric method based on the complexometric indicator murexide (MX), which forms a stable complex with divalent zinc (Zn²⁺) was used (Fig. 1). In the absence of Zn²⁺, MX has a pink color, whereas in the presence of the divalent cation it turns yellow. Pyrophosphate (PPi), released during DNA polymerization, can form a strong complex with zinc, thus displacing the Zn²⁺ cations from MX and inducing a color change of MX from yellow to pink. By mimicking the LAMP reaction components in a test tube, an evident color shift from yellow to pink was observed after PPi addition (Fig. 2A).

In a further embodiment, after an incubation period at 65 °C for 30 minutes, the tubes were opened and murexide (0.5 mM) and ZnCl₂ (2.5 mM) were added to the reaction.

In an embodiment, the sensitivity of Zn-MX (Fig. 2C) with that of phenol red (Fig. 2B) using IVT RNA was compared. It was found that the former performed slightly better than the latter, with a limit of detection of 25 viral copies per reaction (Fig. 2B and C). Like phenol red, Zn-MX showed an evident color difference depending on the presence (pink) or absence (yellow) of LAMP amplification (Fig. 2C). Moreover, the method enabled the clear detection of SARS-CoV-2 in crude saliva samples (Fig. 2D), more efficiently than the phenol red method (Fig. 2E). In another embodiment, the method also enables the detection of the SARS-CoV-2 genetic material in RNA samples extracted from nasopharyngeal samples (Fig. 2F).

In an embodiment, the colorimetric test described in the present disclosure rapidly detects IVT RNA of SARS-CoV-2. The assay also allows for detection of the virus directly from patient's saliva with minimal processing and increased protection of the testing personnel.

The analytical LoD of the SARS-CoV-2 test is sufficient to identify individuals with viral titers high enough to transmit the virus (300-1000 viral copies per µL) [27,33]. This test sensitivity is understood to be adequate for surveillance and screening of the asymptomatic population. The availability of such a testing solution is therefore of great importance, as infectiousness peaks occur before or at the symptom's onset.

In another embodiment, the colorimetric reading described in the present disclosure is independent of changes in the pH of the amplifying reaction, thus eliminating the impact of acidic saliva samples on the test readout. In an embodiment, the method can be safely used in closed systems, such as microfluidic diagnostic cartridges, among others.

In an embodiment, the murexide-zinc colorimetric method was performed with analytical grade reagents. Solutions were prepared in ultrapure grade water (Milli-Q), and samples for in-vitro tests were buffered at pH=7.4 with 20 mM of 3-(N-morpholino)propanesulfonic acid (MOPS) as follows: (3-(N-morpholino)propanesulfonic acid) (MOPS) buffer pH=7.4 at 20 mM, magnesium chloride (MgCl₂) at 47.5 mM, zinc chloride (ZnCl₂) at 47.1 mM, sodium pyrophosphate (Na₄P₂O₇) at 50 mM, ATP at 25 mM, and murexide (MX) at 0.5 mM. The MX solution was prepared immediately before use or otherwise kept frozen.

The following examples further illustrate the present disclosure in detail but are not to be construed to limit the scope thereof. The examples outside the scope of the claims are comparative examples.

**Example 1:** detection ensemble comprising Zinc(II) chloride (CAS 7646-85-7). An aqueous sample of 1 mL (mimicking the components of the LAMP reaction) containing 8 mM of magnesium chloride and 1.4 mM of ATP (as a surrogate of dNTPs) was prepared, buffered at pH=7.4 with 10 mM of MOPS. Addition of 20 µL of murexide (MX) solution at 10 mM to the sample produced a visible pink color, corresponding to the indicator in the free form. Addition of 10 µL of zinc(II) chloride solution at 47.1 mM to the sample, changed its color to orange, indicating a change of the indicator to the complexed form (bound to the metal cation). Finally, the addition of 30 µL of pyrophosphate solution at 50 mM to the sample caused it to turn back to pink, marking the displacement of the indicator from zinc(II) induced by the binding of pyrophosphate to zinc(II) (Fig. 2A).

**Example 2:** detection ensemble comprising Zinc(II) complex of dipicolinic acid (DP, 2,6-Pyridinedicarboxylic acid, CAS 499-83-2). An aqueous sample of 1 mL (mimicking the components of the LAMP reaction) containing 8 mM of magnesium chloride and 1.4 mM of ATP (as a surrogate of dNTPs) was prepared, buffered at pH=7.4 with 10 mM of MOPS. Addition of 20 µL of murexide solution at 10 mM to the sample produced a visible pink color, corresponding to the indicator in the free form. Addition of 30 µL of a solution at 20 mM of the dipicolinic acid complex of zinc(II) (1:1 metal to ligand ratio) changed it to orange, indicating a change of the indicator to the complexed form (bound to the metal cation). Finally, addition of 20 µL of pyrophosphate solution at 50 mM to the sample caused it to change back to pink, indicating the displacement of the indicator from zinc(II) caused by the binding of pyrophosphate to the zinc(II) complex of dipicolinic acid (Fig. 3A and 3B).

**Example 3** Zinc(II) complex of 1,4,7-triazacyclononane (TACN, CAS 4730-54-5). An aqueous sample of 1 mL (mimicking the components of the LAMP reaction) containing 8 mM of magnesium chloride and 1.4 mM of ATP (as a surrogate of dNTP's) was prepared, buffered at pH=7.4 with 10 mM of MOPS. Addition of 20 µL of murexide solution at 10 mM to the sample resulted in a visible pink color, corresponding to the indicator in the free form. Addition of 140 µL of a solution at 23.6 mM of the zinc(II) complex of TACN (1:1 metal to ligand ratio) changed it to orange, indicating a change of the indicator to the complexed form (bound to the metal cation). Finally, addition of 40 µL of pyrophosphate solution at 50 mM to the sample changed it back to pink, marking the displacement of the indicator from zinc(II) caused by the binding of pyrophosphate to the zinc(II) complex of TACN (Fig. 3C and 3D).

**Example 4:** Zinc(II) complex of dipicolylamine (DPA, Bis(2-pyridylmethyl)amine, CAS 1539-42-0). An aqueous sample of 1 mL (mimicking the components of the LAMP reaction) containing 8 mM of magnesium chloride and 1.4 mM of ATP (as a surrogate of dNTP's) was prepared, buffered at pH=7.4 with 10 mM of MOPS. Addition of 20 µL of murexide solution at 10 mM to the sample resulted in a visible pink color, corresponding to the indicator in the free form. Addition of 20 µL of a solution at 24.3 mM of the dipicolylamine complex of zinc(II) (1:1 metal to ligand ratio) changed it to orange, indicating a change of the indicator to the complexed form (bound to the metal cation). Finally, addition of 10 µL of pyrophosphate solution at 50 mM to the sample changed it back to a pink, marking the displacement of the indicator from zinc(II) caused by the binding of pyrophosphate to the zinc(II) complex of dipicolylamine (Fig. 3E and 3F) .

**Example 5:** Zinc(II) complex of N-benzyl-dipicolylamine (BnDPA, N-benzyl-1-(pyridin-2-yl)-N-(pyridin-2-ylmethyl)methanamine, CAS 26082-96-2). An aqueous sample of 1 mL (mimicking the components of the LAMP reaction) containing 8 mM of magnesium chloride and 1.4 mM of ATP (as a surrogate of dNTP's) was prepared, buffered at pH=7.4 with 10 mM of MOPS. Addition of 20 µL of murexide solution at 10 mM to the sample resulted in a visible pink color, corresponding to the indicator in the free form. Addition of 20 µL of a solution at 23 mM of the zinc(II) complex of BnDPA (1:1 metal to ligand ratio) changed it to orange, indicating a change of the indicator to the complexed form (bound to the metal cation). Finally, addition of 20 µL of pyrophosphate solution at 50 mM to the sample changed it back to pink, marking the displacement of the indicator from zinc(II) caused by the binding of pyrophosphate to the zinc(II) complex of BnDPA (Fig. 3H and 3I).

**Example 6:** Zinc(II) complex of N-bromophenol-dipicolylamine (BrPhDPA, 22-((bis(pyridin-2-ylmethyl)amino)methyl)-4-bromophenol, CAS 209747-83-1). An aqueous sample of 1 mL (mimicking the components of the LAMP reaction) containing 8 mM of magnesium chloride and 1.4 mM of ATP (as a surrogate of dNTP's) was prepared, buffered at pH=7.4 with 10 mM of MOPS. Addition of 20 µL of murexide solution at 10 mM to the sample resulted in a visible pink color, corresponding to the indicator in the free form. Addition of 140 µL of a solution at 23 mM of the zinc(II) complex of BrPhDPA (1:1 metal to ligand ratio) changed it to orange, indicating a change of the indicator to the complexed form (bound to the metal cation). Finally, addition of 40 µL of pyrophosphate solution at 50 mM to the sample changed it back to pink, marking the displacement of the indicator from zinc(II) caused by the binding of pyrophosphate to the zinc(II) complex of BrPhDPA (Fig. 3J and 3K).

**Example 7:** Zinc(II) chloride (CAS 7646-85-7) for detection of genetic viral material. Clinical specimens were collected at Hospital das Forças Armadas and then processed in Laboratório de Bromatologia e Defesa Biológica (Unidade Militar Laboratorial de Defesa Biológica e Química). Saliva specimens (~1ml) were self-collected into sterile tubes (50 ml or 1.5 mL). Patients were asked not to eat or drink before testing. NP swab-matched samples were collected in parallel and placed in 3 ml Universal Viral Transport Media system. Tubes containing clinical specimens were decontaminated with an alcohol-based solution and identified. After collection, samples were kept at 4°C for 2-4 days or processed immediately. Samples were inactivated by incubation at 95°C for 5 minutes (NP swabs) or 30 minutes (saliva samples). Salivas were centrifuged at 5000 g for 5 minutes and 200 µL of the supernatant were diluted in TAE 10x (1x, final concentration) and frozen a- - 80°C until analysis. The saliva pellets were also frozen. Total viral RNA was extracted from 140 µl of NP deactivated samples using Viral RNA Mini Kit (QIAGEN) and eluted in 60 µl of RNAse free water, to ensure the RNA elution buffer has no impact of pH in RT-LAMP reactions. To prepare the SARS-CoV-2 RNA standard (IVT RNA), the N gene was amplified from the plasmid 2019-nCoV_N_Positive Control (Integrated DNA Technologies) with a T7-promoter-containing primer (SEQ. ID. No. 1 - 5' - TAATACGACTCACTATAGGatgtctgataatggaccccaaaa - 3') and the reverse primer (SEQ. ID. No. 2 - 5'- ttaggcctgagttgagtcagc-3'), and then the product was *in vitro* transcribed using the HiScribe T7 High Yield RNA Synthesis Kit, NEB), according to the manufacturer's instructions. Template DNA was removed using Turbo DNase (Invitrogen) and RNA was then purified using RNeasy (Qiagen). Standard RNA copy numbers were calculated from concentration measured using Take3 from Epoch from Biotek and confirmed using a Ultrospec2100pro (Amersham Biosciences). SARS-CoV-2 N-gene and an internal control (RNase P) were amplified by RT-PCR, as comparative data, using the TaqMan 2019-nCoV Assay Kit v1 (Termofisher) with TaqMan Fast Virus 1-step Master Mix (Termofisher) and the CFX96 thermocyler (BioRad), according to the manufacturer's instructions. The RT-LAMP reaction was performed in a total volume of 20 µL containing the following components: colorimetric buffer (1.6 µM FIP/BIP primers, 0.4 µM LF/LB primers, 0.2 µM F3/B3 primers Gene N-A [24], 10 mM (NH₄)₂SO₄ (Merck), 50 mM KCI (BDH), 8 mM MgSO₄ (BDH), 0.1% Tween 20, 0.2 mM Phenol Red (Sigma), 1.4 mM each dNTP (NZYTech)), 8 U Bst 2.0 (NEB), 15 U RTx (NEB) and 2µL of saliva or RNA. When the complexometric indicator MX-Zn was used, samples were assembled as described above, but without phenol red. After 30 min, 2µL of 5 mM murexide and 1 µL of 50 mM of ZnCl₂ were added to the reaction, in a post-LAMP workspace. All reactions were performed in a thermocycler (Bio-Rad) at 65°C and pictures were taken at the indicated time points (Fig.2).

**Example 8:** Zinc(II) complex of dipicolylamine (DPA, Bis(2-pyridylmethyl)amine, CAS 1539-42-0) for detection of genetic material of the fungus *Candida albicans* amplified by LAMP. Genomic DNA of *Candida albicans* was isolated using the phenol:chloroform:isoamyl alcohol and glass beads method, precipitated with ethanol resuspended in water and quantified. The LAMP reaction was performed in a total volume of 20 µL containing the following components: 1.6 µM FIP/BIP primers, 0.4 µM LB primers, 0.2 µM F3/B3 primers, 10 mM (NH₄)₂SO₄ (Merck), 50 mM KCI (BDH), 8 mM MgSO₄ (BDH), 0.1% Tween 20, 1.4 mM each dNTP (NZYTech)), 8 U Bst 2.0 (NEB). The genomic DNA was diluted to 2.5x10⁻³ ng /µL and 2 µL were added to the reaction mix. After 30 min at 65 °C in a thermocycler (Eppendorf), 2µL of 5 mM murexide and 6 µL of 24 mM of Zn-DPA were added to the reaction, in a post-LAMP workspace (Fig. 3G). The sequence of the primers used (*ITS2* gene) is available in [35].

**Example 9:** Zinc(II) sulfate for detection of genetic material of the fungus *Candida albicans* amplified by LAMP. Genomic DNA of *Candida albicans* was isolated and amplified as described in Example 8. After 30 min at 65 °C in a thermocycler (Eppendorf), 2µL of 5 mM murexide and 2 µL of 5 mM of ZnSO₄ were added to the reaction, in a post-LAMP workspace (Fig. 4B).

**Example 10:** Zinc(II) chloride (CAS 7646-85-7) for detection of human, plant and bacterial genetic material amplified by LAMP. For detection of the human *POP7* gene, the genetic material was extracted from NP swab fluids as described in Example 7. The LAMP reaction was performed in a total volume of 20 µL containing the following components: 1.6 µM FIP/BIP primers, 0.4 µM LB primers, 0.2 µM F3/B3 primers, 10 mM (NH_{Apipo}SO₄ (Merck), 50 mM KCI (BDH), 8 mM MgSO₄ (BDH), 0.1% Tween 20, 1.4 mM each dNTP (NZYTech)), 15U RTx and 8 U Bst 2.0 (NEB). The genomic DNA was diluted to 2.5x10⁻³ ng /µL and 2 µL were added to the reaction mix. After 30 min at 65 °C in a thermocycler (Eppendorf) 2µL of 5 mM murexide and 2 µL of 47 mM of ZnCl₂ were added to the reaction, in a post-LAMP workspace (Fig.5A). The sequence of the primers used *(POP7* gene) is available in [36]. For detection of the rice (*Oryza sativa*) *PLD* gene, the genetic material was extracted from *Oryza sativa nipponbare.* The LAMP reaction was performed in a total volume of 20 µL containing the following components: 1.6 µM FIP/BIP primers, 0.4 µM LF/LB primers, 0.2 µM F3/B3 primers, 10 mM (NH₄)₂SO₄ (Merck), 50 mM KCI (BDH), 8 mM MgSO₄ (BDH), 0.1% Tween 20, 1.4 mM each dNTP (NZYTech), 8 U Bst 2.0 (NEB). The genomic DNA was diluted to 2.5x10⁻³ ng /µL and 2 µL were added to the reaction mix. After 30 min at 65 °C in a thermocycler (Eppendorf) 2µL of 5 mM murexide and 2 µL of 47 mM of ZnCl₂ were added to the reaction, in a post-LAMP workspace (Fig. 5B). The sequence of the primers used (*PLD* gene) is available in [37]. For detection of the DNA from the bacterium *Clostridium difficile,* the genomic DNA was isolated using DNA from bacterial *Clostridium difficile* was extracted by phenol:chloroform:isoamyl alcohol after digestion with mutanolysin in the presence of detergent, RNase and proteinase K. After precipitation with ethanol it was dialysed against water. The LAMP reaction was performed in a total volume of 20 µL containing the following components for the *C. difficile* mix: 1.6 µM FIP/BIP primers, 0.4 µM LF/LB primers, 0.2 µM F3/B3 primers, 10 mM (NH₄)₂SO₄ (Merck), 50 mM KCI (BDH), 8 mM MgSO₄ (BDH), 0.1% Tween 20, 1.4 mM each dNTP (NZYTech), 8 U Bst 2.0 (NEB) and 2 µL of DNA. After 30 min at 65 °C in a thermocycler (Eppendorf) 2µL of 5 mM Murexide and 2 µL of 47 mM of ZnCl₂ were added to the reaction, in a post-LAMP workspace (Fig. 5C). The sequence of the primers used (*TcdB* gene) is available in [38].

**Example 11:** Zinc(II) complex of N-bromophenol-dipicolylamine (BrPhDPA, 2-((bis(pyridin-2-ylmethyl)amino)methyl)-4-bromophenol, CAS 209747-83-1) for detection of genetic material of the fungus *Candida albicans* amplified by LAMP. Genomic DNA of *Candida albicans* was isolated using the phenol:chloroform:isoamyl alcohol and glass beads method, precipitated with ethanol resuspended in water and quantified. The LAMP reaction was performed in a total volume of 20 µL containing the following components: 1.6 µM FIP/BIP primers, 0.4 µM LB primers, 0.2 µM F3/B3 primers, 1x Isothermal Amplification Buffer (NEB), 6mM MgSO₄, 1.4 mM each dNTP (NZYTech)), 8 U Bst 2.0 (NEB). The genomic DNA was diluted to 2.5 ng /µL and 2 µL were added to the reaction mix. After 30 min at 65 °C in a thermocycler (Eppendorf), 2µL of 5 mM murexide and 2 µL of 23 mM of Zn- BrPhDPA were added to the reaction, in a post-LAMP workspace (Fig. 3L). The sequence of the primers used (*ITS2* gene) is available in [35].

**Example 12:** Zinc(II) Chloride (ZnCl₂) for detection of a sequence of genetic material - a bacterial plasmid (pRS314 or pRS416) amplified by PCR using Taq polymerase. µM primer forward (SEQ ID No. 3 - 5' GTTTTCCCAGTCACGACGTT 3'), 0.2 µM primer reverse (SEQ ID No.4 - 5' TTATGCTTCCGGCTCCTATG 3') and 0.05 U of NZYTaq DNA Polymerase. 200ng of plasmid was used as template for each PCR. The reaction mixture was subjected to 95 °C for 3min, and 35 cycles of 94 °C, 30 sec, 55 °C, 30 sec and 72 °C 20 sec, with the final extension at 72 °C for 5 min in a thermo-cycler (Biometra). After the reaction 2µL of 5mM murexide and 2µL of 5mM ZnCl₂ were added to the reaction. Amplification products were also analysed by 2% agarose gel electrophoresis (Fig.6A).

**Example 13:** Zinc(II) complex of dipicolinic acid (DP, 2,6-Pyridinedicarboxylic acid, CAS 499-83-2) for detection of a sequence of genetic material - a bacterial plasmid (pRS314 or pRS416) amplified by PCR using Taq polymerase. The PCR reaction was performed in a total volume of 20 µL containing the following components: NZYTech reaction buffer 1x, 2.5 mM MgCl₂, 0,4 mM dNTPs, 0.2 µM primer forward (SEQ ID No. 3 - 5' GTTTTCCCAGTCACGACGTT 3'), 0.2 µM primer reverse (SEQ ID No. 4 - 5' TTATGCTTCCGGCTCCTATG 3') and 0.05 U of NZYTaq DNA Polymerase. 200 ng of plasmid was used as template for each PCR. The reaction mixture was subjected to 95 °C for 3min, and 35 cycles of 94 °C, 30 sec, 55 °C, 30 sec and 72 °C 20 sec, with the final extension at 72 °C for 5 min in a thermo-cycler (Biometra). After the reaction 2µL of 5 mM murexide and 2µL of 10mM Zn-DP were added to the reaction (Fig.6B).

**Example 14:** Zinc(II) complex of dipicolylamine (DPA, Bis(2-pyridylmethyl)amine, CAS 1539-42-0) for detection of a sequence of genetic material-- a bacterial plasmid (pRS314 or pRS416) amplified by PCR using Taq polymerase. The PCR reaction was performed in a total volume of 20 µL containing the following components: NZYTech reaction buffer 1x, 2.5 mM MgCl₂, 0.4 mM dNTPs, 0.2 µM primer forward (SEQ ID No. 3 - 5' GTTTTCCCAGTCACGACGTT 3'), 0.2 µM primer reverse (SEQ ID No. 4 - 5' TTATGCTTCCGGCTCCTATG 3') and 0.05 U of NZYTaq DNA Polymerase. 200ng of plasmid was used as template for each PCR. The reaction mixture was subjected to 95 °C for 3min, and 35 cycles of 94 °C, 30 sec, 55 °C, 30 sec and 72 °C 20 sec, with the final extension at 72 °C for 5 min in a thermo-cycler (Biometra). After the reaction 2 µL of 5mM murexide and 2 µL of 10 mM Zn-DPA were added to the reaction (Fig.6C).

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above described embodiments are combinable.

The following claims further set out particular embodiments of the disclosure.

### References:

1. Nagamine K, Hase T, Notomi T (2002) Accelerated reaction by loop-mediated isothermal amplification using loop primers. Mol Cell Probes 16: 223-229.
2. Notomi T, Okayama H, Masubuchi H, Yonekawa T, Watanabe K, et al. (2000) Loop-mediated isothermal amplification of DNA. Nucleic Acids Res 28: E63.
3. Tomita N, Mori Y, Kanda H, Notomi T (2008) Loop-mediated isothermal amplification (LAMP) of gene sequences and simple visual detection of products. Nat Protoc 3: 877-882.
4. Ahn SJ, Baek YH, Lloren KKS, Choi WS, Jeong JH, et al. (2019) Rapid and simple colorimetric detection of multiple influenza viruses infecting humans using a reverse transcriptional loop-mediated isothermal amplification (RT-LAMP) diagnostic platform. BMC Infect Dis 19: 676.
5. Bhadra S, Jiang YS, Kumar MR, Johnson RF, Hensley LE, et al. (2015) Real-time sequence-validated loop-mediated isothermal amplification assays for detection of Middle East respiratory syndrome coronavirus (MERS-CoV). PLoS One 10: e0123126.
6. Hong TC, Mai QL, Cuong DV, Parida M, Minekawa H, et al. (2004) Development and evaluation of a novel loop-mediated isothermal amplification method for rapid detection of severe acute respiratory syndrome coronavirus. J Clin Microbiol 42: 1956-1961.
7. Jayawardena S, Cheung CY, Barr I, Chan KH, Chen H, et al. (2007) Loop-mediated isothermal amplification for influenza A (H5N1) virus. Emerg Infect Dis 13: 899-901.
8. Lee SH, Baek YH, Kim YH, Choi YK, Song MS, et al. (2016) One-Pot Reverse Transcriptional Loop-Mediated Isothermal Amplification (RT-LAMP) for Detecting MERS-CoV. Front Microbiol 7: 2166.
9. Thompson DL, Y. (2020) Recent progress in RT-LAMP enabled COVID-19 detection . Sensors and Actuators Reports 2.
10. Quyen TL, Ngo TA, Bang DD, Madsen M, Wolff A (2019) Classification of Multiple DNA Dyes Based on Inhibition Effects on Real-Time Loop-Mediated Isothermal Amplification (LAMP): Prospect for Point of Care Setting. Front Microbiol 10: 2234.
11. Goto M, Honda E, Ogura A, Nomoto A, Hanaki K (2009) Colorimetric detection of loop-mediated isothermal amplification reaction by using hydroxy naphthol blue. Biotechniques 46: 167-172.
12. Tanner NA, Zhang Y, Evans TC, Jr. (2015) Visual detection of isothermal nucleic acid amplification using pH-sensitive dyes. Biotechniques 58: 59-68.
13. Fischbach J, Xander NC, Frohme M, Glokler JF (2015) Shining a light on LAMP assays--a comparison of LAMP visualization methods including the novel use of berberine. Biotechniques 58: 189-194.
14. Lamb LE, Bartolone SN, Ward E, Chancellor MB (2020) Rapid detection of novel coronavirus/Severe Acute Respiratory Syndrome Coronavirus 2 (SARS-CoV-2) by reverse transcription-loop-mediated isothermal amplification. PLoS One 15: e0234682.
15. Park GS, Ku K, Baek SH, Kim SJ, Kim SI, et al. (2020) Development of Reverse Transcription Loop-Mediated Isothermal Amplification Assays Targeting Severe Acute Respiratory Syndrome Coronavirus 2 (SARS-CoV-2). J Mol Diagn 22: 729-735.
16. Anahtar MN, McGrath GE, Rabe BA, Tanner NA, White BA, et al. (2020) Clinical assessment and validation of a rapid and sensitive SARS-CoV-2 test using reverse-transcription loop-mediated isothermal amplification. medRxiv: 2020.2005.2012.20095638.
17. Buck MD, Poirier EZ, Cardoso A, Frederico B, Canton J, et al. (2020) Standard operating procedures for SARS-CoV-2 detection by a clinical diagnostic RT-LAMP assay. medRxiv: 2020.2006.2029.20142430.
18. Butler DJ, Mozsary C, Meydan C, Danko D, Foox J, et al. (2020) Shotgun Transcriptome and Isothermal Profiling of SARS-CoV-2 Infection Reveals Unique Host Responses, Viral Diversification, and Drug Interactions. bioRxiv: 2020.2004.2020.048066.
19. Dao Thi VL, Herbst K, Boerner K, Meurer M, Kremer LP, et al. (2020) A colorimetric RT-LAMP assay and LAMP-sequencing for detecting SARS-CoV-2 RNA in clinical samples. Sci Transl Med 12.
20. Huang WE, Lim B, Hsu CC, Xiong D, Wu W, et al. (2020) RT-LAMP for rapid diagnosis of coronavirus SARS-CoV-2. Microb Biotechnol 13: 950-961.
21. Kellner MJ, Ross JJ, Schnabl J, Dekens MPS, Heinen R, et al. (2020) A rapid, highly sensitive and open-access SARS-CoV-2 detection assay for laboratory and home testing. bioRxiv: 2020.2006.2023.166397.
22. Rabe BA, Cepko C (2020) SARS-CoV-2 detection using isothermal amplification and a rapid, inexpensive protocol for sample inactivation and purification. Proc Natl Acad Sci U S A 117: 24450-24458.
23. Yu L, Wu S, Hao X, Dong X, Mao L, et al. (2020) Rapid Detection of COVID-19 Coronavirus Using a Reverse Transcriptional Loop-Mediated Isothermal Amplification (RT-LAMP) Diagnostic Platform. Clin Chem 66: 975-977.
24. Zhang Y, Odiwuor N, Xiong J, Sun L, Nyaruaba RO, et al. (2020) Rapid Molecular Detection of SARS-CoV-2 (COVID-19) Virus RNA Using Colorimetric LAMP. medRxiv: 2020.2002.2026.20028373.
25. To KK, Tsang OT, Leung WS, Tam AR, Wu TC, et al. (2020) Temporal profiles of viral load in posterior oropharyngeal saliva samples and serum antibody responses during infection by SARS-CoV-2: an observational cohort study. Lancet Infect Dis 20: 565-574.
26. To KK, Tsang OT, Yip CC, Chan KH, Wu TC, et al. (2020) Consistent Detection of 2019 Novel Coronavirus in Saliva. Clin Infect Dis 71: 841-843.
27. Wyllie AL, Fournier J, Casanovas-Massana A, Campbell M, Tokuyama M, et al. (2020) Saliva or Nasopharyngeal Swab Specimens for Detection of SARS-CoV-2. N Engl J Med 383: 1283-1286.
28. Dudley DM, Newman CM, Weiler AM, Ramuta MD, Shortreed CG, et al. (2020) Optimizing direct RT-LAMP to detect transmissible SARS-CoV-2 from primary nasopharyngeal swab and saliva patient samples. medRxiv: 2020.2008.2030.20184796.
29. Lalli MA, Langmade SJ, Chen X, Fronick CC, Sawyer CS, et al. (2020) Rapid and extraction-free detection of SARS-CoV-2 from saliva with colorimetric LAMP. medRxiv: 2020.2005.2007.20093542.
30. Ranoa DRE, Holland RL, Alnaji FG, Green KJ, Wang L, et al. (2020) Saliva-Based Molecular Testing for SARS-CoV-2 that Bypasses RNA Extraction. bioRxiv: 2020.2006.2018.159434.
31. Yang Q, Meyerson NR, Clark SK, Paige CL, Fattor WT, et al. (2020) Saliva TwoStep: An RT-LAMP saliva test for SARS-CoV-2 and its assessment in a large population. medRxiv: 2020.2007.2016.20150250.
32. Kim D, Lee JY, Yang JS, Kim JW, Kim VN, et al. (2020) The Architecture of SARS-CoV-2 Transcriptome. Cell 181: 914-921 e910.
33. Amaral C, Antunes W, Moe E, Duarte AG, Lima LMP, et al. (2021) A molecular test based on RT-LAMP for rapid, sensitive and inexpensive colorimetric detection of SARS-CoV-2 in clinical samples. Scientific reports 11: 16430-16430.
34. Yaffe H, Buxdorf K, Shapira I, Ein-Gedi S, Moyal-Ben Zvi M, et al. (2012) LogSpin: a simple, economical and fast method for RNA isolation from infected or healthy plants and other eukaryotic tissues. BMC Research Notes 5: 45.
35. Fallahi S, Babaei M, Rostami A, Mirahmadi H, Arab-Mazar Z, et al. (2020) Diagnosis of Candida albicans: conventional diagnostic methods compared to the loop-mediated isothermal amplification (LAMP) assay. Archives of Microbiology 202: 275-282.
36. Curtis KA, Morrison D, Rudolph DL, Shankar A, Bloomfield LSP, et al. (2018) A multiplexed RT-LAMP assay for detection of group M HIV-1 in plasma or whole blood. Journal of Virological Methods 255: 91-97.
37. Narushima J, Kimata S, Soga K, Sugano Y, Kishine M, et al. (2020) Rapid DNA template preparation directly from a rice sample without purification for loop-mediated isothermal amplification (LAMP) of rice genes. Bioscience, Biotechnology, and Biochemistry 84: 670-677.
38. Kato H, Yokoyama T, Kato H, Arakawa Y (2005) Rapid and Simple Method for Detecting the Toxin B Gene of Clostridium difficile in Stool Specimens by Loop-Mediated Isothermal Amplification. Journal of Clinical Microbiology 43: 6108-6112.

PCR PRIMER SEQUENCES (5'-3')
   SEQ ID No. 1 - PCR primers gene N and in vitro RNA synthesis : TAATACGACTCACTATAGGatgtctgataatggaccccaaaa
   SEQ ID No. 2 - PCR primer gene N : Ttaggcctgagttgagtcagc
   SEQ ID No. 3 - PCR Primer PRS416 Fwd: GTTTTCCCAGTCACGACGTT
   SEQ ID No. 4 - PCR Primer PRS416 Rev: TTATGCTTCCGGCTCCTATG
LAMP PRIMER SEQUENCES (5'-3')
SARSCoV-2
   SEQ ID No. 5 - RT-LAMP Primer GeneN-A-F3 : TGGCTACTACCGAAGAGCT 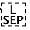
   SEQ ID No. 6 - RT-LAMP Primer GeneN-A-B3- : TGCAGCATTGTTAGCAGGAT
   SEQ ID No. 7 - RT-LAMP Primer GeneN-A-FIP :
      TCTGGCCCAGTTCCTAGGTAGTCCAGACGAATTCGTGGTGG
   SEQ ID No. 8 - RT-LAMP Primer GeneN-A-BIP :
      AGACGGCATCATATGGGTTGCACGGGTGCCAATGTGATCT
   SEQ ID No. 9 - RT-LAMP Primer GeneN-A-LF: GGACTGAGATCTTTCATTTTACCGT
   SEQ ID No. 10 - RT-LAMP Primer GeneN-A-LB : ACTGAGGGAGCCTTGAATACA
Candida albicans
   SEQ ID No. 11 - LAMP Primer Ca-F3: TCTGGTATCCGGAGGGC
   SEQ ID No. 12 - LAMP Primer Ca-B3: AGTCCTACCTGATTTGAGGT
   SEQ ID No. 13 - LAMP Primer Ca-FIP: CTACCGTCTTTCAAGCAAACCCATGAGCGTCGTTTCTCCCT
Homo sapiens
   SEQ ID No. 14 - LAMP Primer RNaseP POP7 F3: TTGATGAGCTGGAGCCA
   SEQ ID No. 15 - LAMP Primer RNaseP POP7 B3: CACCCTCAATGCAGAGTC
   SEQ ID No. 16 - LAMP Primer RNaseP POP7 LF : ATGTGGATGGCTGAGTTGTT
   SEQ ID No. 17 - LAMP Primer RNaseP POP7 LB : CATGCTGAGTACTGGACCTC
   SEQ ID No. 18 - LAMP Primer RNaseP POP7 FIP : GTGTGACCCTGAAGACTCGGTTTTAGCCACTGACTCGGATC
   SEQ ID No. 19 - LAMP Primer RNaseP POP7 BIP :
      CCTCCGTGATATGGCTCTTCGTTTTTTTCTTACATGGCTCTGGTC
Oryza sativa
   SEQ ID No. 20 - LAMP Primer Os-FIP : AACACTCCAGGCCTCACCGTGGCCGACCTCAT TATTCCG
   SEQ ID No. 21 - LAMP Primer Os-BIP : GTTCCGGTCCATCGATGGCTGCAGCCTCTGGAGTGCTA
   SEQ ID No. 22 - LAMP Primer Os-F3 : GACCTCCTCCTAGACCTCAA
   SEQ ID No. 23 - LAMP Primer Os-B3 : TGACAAGGCCTGATCTTGC
   SEQ ID No. 24 - LAMP Primer Os-LF : GGAACATCACCGGAGACGG
   SEQ ID No. 25 - LAMP Primer Os-LB : GCGGCCTGCTTTGGCTT
Clostridium difficile
   SEQ ID No. 26 - LAMP Primer HK101-F3 : GTATCAACTGCATTAGATGAAAC
   SEQ ID No. 27 - LAMP Primer HK101-B3: CCAAAGATGAAGTAATGATTGC
   SEQ ID No. 28 - LAMP Primer HK101-FIP :
      CTGCACCTAAACTTACACCATCTATCTTCCTACATTATCTGAAGGATT
   SEQ ID No. 29 - LAMP Primer HK101-BIP :
      GAGCTAAGTGAAACGAGTGACCCGCTGTTGTTAAATTTACTGCC
   SEQ ID No. 30 - LAMP Primer HK101-FL : AATAGTTGCAATTATAGG
   SEQ ID No. 31 - LAMP Primer HK101-BL: AGACAAGAAATAGAAGCTAAGATAGG

## Claims

1. A colorimetric method for a detection of an amplified genetic material in a biological sample, comprising the following steps:
- amplifying the genetic material of the biological sample to obtain a solution of amplified genetic material;
- adding a detection ensemble comprising murexide and zinc(II) chloride to the solution, wherein the concentration of the zinc(II) chloride ranges from 0.4 to 3 mM; and
- evaluating the color change.

2. The method according to the previous claim wherein the amplification step comprises a polymerization step with production of pyrophosphate.

3. Method according to any of the previous claims wherein the zinc(II) chloride is a complex with an organic ligand selected from a list comprising: dipicolinic acid, dipicolylamine, dipicolylamine derivatives, polyamine compounds, or mixtures thereof.

4. The method according to any of the previous claims wherein the genetic material of the biological sample is selected from viral, bacterial, fungal, protozoal parasite, plant genetic material, or human genetic material.

5. The method according to any of the previous claims wherein the genetic material of the biological sample is viral genetic material, preferably from a coronavirus, influenza virus, or zika virus, more preferably from SARS-CoV-2.

6. The method according to any of the previous claims wherein the amplification step comprises a polymerase addition wherein the polymerase is selected from a list consisting of: Bst1, Bst2, Bst3, Taq, Pfu, Pfx, mixtures thereof.

7. The method according to any of the previous claims wherein the biological sample is a saliva, nasopharyngeal sample, blood, or plant tissues, preferably saliva.

8. The method according to any of the previous claims wherein the amplifying step is an isothermal amplification, preferably wherein the amplifying step is performed by loop-mediated isothermal amplification reaction (LAMP), more preferably wherein the amplifying step is performed by reverse transcription loop-mediated isothermal amplification reaction (RT-LAMP); and/or wherein the amplifying step is performed by polymerase chain reaction (PCR).

9. The method according to any of the previous claims wherein the murexide concentration ranges from 0.1 to 1 mM, preferably from 0.3 to 0.7 mM, more preferably from 0.4-0.5 mM.

10. The method according to any of the previous claims wherein the concentration of zinc(II) chloride ranges from 1 to 3 mM; preferably wherein the concentration of zinc(II) chloride ranges from 1.5 to 2.5 mM.

11. The method according to any of the previous claims further comprising an RNA or DNA extraction step before the amplification step, and/or further comprising a step of incubating the solution of amplified genetic material.

12. The method according to any of the previous claims wherein the limit of detection is 20 to 100 viral copies per reaction, preferably 25 viral copies per reaction.

13. Use of a kit for detection of an amplified genetic material in a biological sample by the method as described in any of the previous claims wherein the kit comprises murexide and zinc(II) chloride as an indicator for detection of an amplified genetic material in a biological sample, wherein the concentration of the zinc(II) chloride ranges from 0.4 to 3 mM.

14. Use of murexide and zinc(II) chloride as an indicator for detection of an amplified genetic material in a biological sample, wherein the concentration of the zinc(II) chloride ranges from 0.4 to 3 mM.

15. Use of a microfluidic diagnostic cartridge or a lab-on-chip device for detection of an amplified genetic material in a biological sample by a colorimetric method as described in any of the previous claims, wherein the microfluidic diagnostic cartridge or a lab-on-chip device comprises murexide and zinc(II) chloride as an indicator for detection of an amplified genetic material in a biological sample, wherein the concentration of the zinc(II) chloride ranges from 0.4 to 3 mM.

## Patentansprüche

1. Ein kolorimeres Verfahren zum Nachweis eines amplifizierten genetischen Materials in einer biologischen Probe, umfassend die folgenden Schritte:
- Amplifizieren des genetischen Materials der biologischen Probe, um eine Lösung aus amplifiziertem genetischem Material zu erhalten;
- Zugeben einer Nachweiskomponente, umfassend Murexid und Zink(II)-chlorid, zu der Lösung, wobei die Konzentration des Zink(II)-chlorids im Bereich von 0,4 bis 3 mM liegt; und
- Bewerten der Farbveränderung.

2. Das Verfahren nach dem vorangehenden Anspruch, wobei der Schritt der Amplifikation einen Schritt der Polymerisation mit Herstellung von Pyrophosphat umfasst.

3. Verfahren nach einem der vorangehenden Ansprüche, wobei das Zink(II)-chlorid ein Komplex mit einem organischen Liganden ist, ausgewählt aus einer Liste umfassend: Dipicolinsäure, Dipicolylamin, Dipicolylaminderivate, Polyaminverbindungen oder Mischungen davon.

4. Das Verfahren nach einem der vorangehenden Ansprüche, wobei das genetische Material der biologischen Probe aus viralem, bakteriellem, pilzlichem, protozoisch parasitärem, pflanzlichem genetischem Material oder humanem genetischem Material ausgewählt ist.

5. Das Verfahren nach einem der vorangehenden Ansprüche, wobei das genetische Material der biologischen Probe virales genetisches Material ist, bevorzugt von einem Coronavirus, Influenzavirus oder Zika-Virus, besonders bevorzugt von SARS-CoV-2.

6. Das Verfahren nach einem der vorangehenden Ansprüche, wobei der Schritt der Amplifikation eine Zugabe einer Polymerase umfasst, wobei die Polymerase aus einer Liste ausgewählt ist, bestehend aus: Bst1, Bst2, Bst3, Taq, Pfu, Pfx, Mischungen davon.

7. Das Verfahren nach einem der vorangehenden Ansprüche, wobei die biologische Probe Speichel, eine nasopharyngeale Probe, Blut oder pflanzliches Gewebe ist, bevorzugt Speichel.

8. Das Verfahren nach einem der vorangehenden Ansprüche, wobei der Schritt der Amplifikation eine isotherme Amplifikation ist, wobei der Schritt der Amplifikation bevorzugt durch eine Schleifen-vermittelte isotherme Amplifikationsreaktion (LAMP) durchgeführt wird, wobei der Schritt der Amplifikation besonders bevorzugt durch eine Schleifen-vermittelte isotherme Amplifikationsreaktion mit reverser Transkriptase (RT-LAMP) durchgeführt wird; und/oder wobei der Schritt der Amplifikation durch eine Polymerase-Kettenreaktion (PCR) durchgeführt wird.

9. Das Verfahren nach einem der vorangehenden Ansprüche, wobei die Murexidkonzentration im Bereich von 0,1 bis 1 mM, bevorzugt von 0,3 bis 0,7 mM, besonders bevorzugt von 0,4-0,5 mM liegt.

10. Das Verfahren nach einem der vorangehenden Ansprüche, wobei die Konzentration von Zink(II)-chlorid im Bereich von 1 bis 3 mM liegt; wobei die Konzentration von Zink(II)-chlorid bevorzugt im Bereich von 1,5 bis 2,5 mM liegt.

11. Das Verfahren nach einem der vorangehenden Ansprüche, ferner umfassend einen Schritt der RNA- oder DNA-Extraktion vor dem Schritt der Amplifikation und/oder ferner umfassend einen Schritt der Inkubation der Lösung des amplifizierten genetischen Materials.

12. Das Verfahren nach einem der vorangehenden Ansprüche, wobei die Nachweisgrenze 20 bis 100 virale Kopien je Reaktion beträgt, bevorzugt 25 virale Kopien je Reaktion.

13. Verwendung eines Kits zum Nachweis eines amplifizierten genetischen Materials in einer biologischen Probe durch das in einem der vorangehenden Ansprüche beschriebene Verfahren, wobei das Kit Murexid und Zink(II)-chlorid als Indikator zum Nachweis eines amplifizierten genetischen Materials in einer biologischen Probe umfasst, wobei die Konzentration des Zink(II)-chlorids im Bereich von 0,4 bis 3 mM liegt.

14. Verwendung von Murexid und Zink(II)-chlorid als Indikator zum Nachweis eines amplifizierten genetischen Materials in einer biologischen Probe, wobei die Konzentration des Zink(II)-chlorids im Bereich von 0,4 bis 3 mM liegt.

15. Verwendung einer mikrofluidischen diagnostischen Kartusche oder einer Lab-on-Chip-Vorrichtung zum Nachweis eines amplifizierten genetischen Materials in einer biologischen Probe durch ein kolorimetrisches Verfahren, wie in einem der vorangehenden Ansprüche beschrieben, wobei die mikrofluidische diagnostische Kartusche oder eine Lab-on-Chip-Vorrichtung Murexid und Zink(II)-chlorid als Indikator zum Nachweis eines amplifizierten genetischen Materials in einer biologischen Probe umfasst, wobei die Konzentration des Zink(II)-chlorids im Bereich von 0,4 bis 3 mM liegt.

## Revendications

1. Un procédé colorimétrique pour la détection d'un matériel génétique amplifié dans un échantillon biologique, comprenant les étapes suivantes :
- amplifier le matériel génétique de l'échantillon biologique pour obtenir une solution de matériel génétique amplifié ;
- ajouter un ensemble de détection comprenant du murexide et du chlorure de zinc(II) à la solution, dans lequel la concentration en chlorure de zinc(II) varie de 0,4 à 3 mM ; et
- évaluer le changement de couleur.

2. Le procédé selon la revendication précédente dans lequel l'étape d'amplification comprend une étape de polymérisation avec la production de pyrophosphate.

3. Procédé selon l'une quelconque des revendications précédentes dans lequel le chlorure de zinc(II) est un complexe avec un ligand organique choisi parmi une liste comprenant : de l'acide dipicolinique, de la dipicolylamine, des dérivés de dipicolylamine, des composés polyamines, ou des mélanges de ceux-ci.

4. Le procédé selon l'une quelconque des revendications précédentes dans lequel le matériel génétique de l'échantillon biologique est choisi parmi du matériel génétique viral, bactérien, fongique, de parasite protozoaire, ou végétal, ou du matériel génétique humain.

5. Le procédé selon l'une quelconque des revendications précédentes dans lequel le matériel génétique de l'échantillon biologique est du matériel génétique viral, préférablement issu d'un coronavirus, d'un virus de l'influenza, ou d'un virus Zika, plus préférablement du SARS-CoV-2.

6. Le procédé selon l'une quelconque des revendications précédentes dans lequel l'étape d'amplification comprend un ajout de polymérase dans lequel la polymérase est choisie parmi une liste consistant en : Bst1, Bst2, Bst3, Taq, Pfu, Pfx, ou des mélanges de ceux-ci.

7. Le procédé selon l'une quelconque des revendications précédentes dans lequel l'échantillon biologique est de la salive, un échantillon nasopharyngé, du sang, ou des tissus végétaux, préférablement de la salive.

8. Le procédé selon l'une quelconque des revendications précédentes dans lequel l'étape d'amplification est une amplification isotherme, préférablement dans lequel l'étape d'amplification est exécutée par réaction d'amplification isotherme médiée par boucle (LAMP), plus préférablement dans lequel l'étape d'amplification est exécutée par réaction d'amplification isotherme médiée par boucle à transcription inverse (RT-LAMP) ; et/ou dans lequel l'étape d'amplification est exécutée par réaction en chaîne par polymérase (PCR).

9. Le procédé selon l'une quelconque des revendications précédentes dans lequel la concentration en murexide varie de 0,1 à 1 mM, préférablement de 0,3 à 0,7 mM, plus préférablement de 0,4 à 0,5 mM.

10. Le procédé selon l'une quelconque des revendications précédentes dans lequel la concentration en chlorure de zinc(II) varie de 1 à 3 mM ; préférablement dans lequel la concentration en chlorure de zinc(II) varie de 1,5 à 2,5 mM.

11. Le procédé selon l'une quelconque des revendications précédentes comprenant également une étape d'extraction d'ARN ou d'ADN avant l'étape d'amplification, et/ou comprenant également une étape d'incubation de la solution de matériel génétique amplifié.

12. Le procédé selon l'une quelconque des revendications précédentes dans lequel la limite de détection est de 20 à 100 copies virales par réaction, préférablement 25 copies virales par réaction.

13. Utilisation d'un kit de détection d'un matériel génétique amplifié dans un échantillon biologique à l'aide du procédé tel que décrit dans l'une quelconque des revendications précédentes dans laquelle le kit comprend du murexide et du chlorure de zinc(II) en tant qu'indicateur pour la détection d'un matériel génétique amplifié dans un échantillon biologique, dans lequel la concentration en chlorure de zinc(II) varie de 0,4 à 3 mM.

14. Utilisation de murexide et de chlorure de zinc(II) en tant qu'indicateur pour la détection d'un matériel génétique amplifié dans un échantillon biologique, dans lequel la concentration en chlorure de zinc(II) varie de 0,4 à 3 mM.

15. Utilisation d'une cartouche de diagnostic microfluidique ou d'un laboratoire sur puce pour la détection d'un matériel génétique amplifié dans un échantillon biologique à l'aide d'un procédé colorimétrique tel que décrit dans l'une quelconque des revendications précédentes, dans laquelle la cartouche de diagnostic microfluidique ou un laboratoire sur puce comprennent du murexide et du chlorure de zinc(II) en tant qu'indicateur pour la détection d'un matériel génétique amplifié dans un échantillon biologique, dans lequel la concentration en chlorure de zinc(II) varie de 0,4 à 3 mM.
